# EUROPEAN PATENT APPLICATION

(11) **EP 2 098 596 A1**
(43) Date of publication of application: **09.09.2009**
(21) Application number: 08075175.3
(22) Date of filing: 06.03.2008
(51) Int. Cl.: C12P 5/02, C12P 7/06, C12F 3/10

(54) **Method and installation for producing electricity and conversion products, such as ethanol**

(71) Applicant: Wageningen Universiteit, 6703 HD Wageningen (NL); Agrotechnology and Food Innovations B.V., 6708 PD Wageningen (NL)
(72) Inventor: Sanders, Johan P.M., 9722 WL Groningen (NL); Meesters, Koen P.H., 3817 DK Amersfoort (NL)
(74) Representative: Prins, Hendrik Willem

(57) **Abstract**

The invention relates to a method for the production of electricity and conversion products, such as ethanol, comprising the steps of:
i) separating a starch source into a starch-rich fraction and a residue fraction;
ii) heating of the starch-rich fraction for the gelling of starch;
iii) releasing of the gelled starch from the starch-rich fraction;
iva) enzymatically converting the gelled starch into sugars;
ivb) converting the sugars into conversion products by fermentation;
v) processing conversion products from the conversion medium;
vi) producing biogas from the residue fraction;
vii) producing electricity and heat from the biogas and/or the residue fraction using heat force coupling; and
viii) using heat produced for one or more of the steps i) to vi).

## Description

The present invention relates to a method and a device for producing electricity and ethanol.

Presently, there is a growing demand for alternatives for fossilized fuels, and particularly for the production of electricity and biofuel. A possible strategy is to use renewable energy sources. For producing electricity and biofuel from renewable energy sources in an as much as possible economially feasible way, it is essential to optimally use these environmental friendly energy sources and to produce, besides electricity, also other valuable products, in the present case ethanol. For an optimal use of renewable energy sources, it is important to use heat coupling, providing electricity or mechanical strength, and, simultaneously, usable thermal energy. This would greatly improve the use of the renewable energy sources.

The present invention is based on the observation that through an optimal use of a starch source, in combination with heat power coupling, a method, and device, for producing electricity and conversion products is provided meeting the above objects. The optimal use of the starch source comprises an adequate separation of the starch source in a starch-rich fraction and a residual fraction. The starch-rich fraction is used for the preparation of microbiological conversion products such as ethanol, butanol, citric acid, lactic acid and derivative products like calcium lactate. The residue fraction is directly used for producing electricity and heat as also indirectly through biogas. The heat generated by the heat force coupling is used for processing and reprocessing of the starch source into starch and conversion products, for producing biogas from the residue fraction, and for a variety of recycling flows.

Therefore, the present invention provides a method for producing electricity and conversion products, such as ethanol, comprising the steps of:
i) separating a starch source into a starch-rich fraction and a residue fraction;
ii) heating the starch-rich fraction for the gelling of starch;
iii) releasing the gelled starch from the starch-rich fraction;
iva) enzymaticaly converting the gelled starch into sugars;
ivb) converting, through fermentation, the sugars into conversion products;
v) further processing the conversion products from the conversion medium;
vi) producing biogas from the residue fraction;
vii) generating, trough heat force coupling, electricity and heat from biogas and/or the residue fraction; and
viii) using the generated heat in one or more of the steps i) to vi).

The starch source is selected in such way that, with relative ease, and preferably using known techniques, a starch source, such as for example a starch storage organ of a plant, such as a bead, a nodule, a root, or parts thereof, can be separated into a starch fraction and a residue fraction. The provided starch-rich fraction, being preferably a bead fraction, additionally and advantageously provides that starch from its natural source is enclosed in a biological casing such as a starch storage organ. It is especially this biological casing which is gelled by heating the starch and, therefore, it will be more available for enzymatic conversion as compared to raw starch. It will be apparent that for the heating which is used for gelling the starch, use is made of the thermal heat derived from the heat force coupling. This provides a useful use of available heat with a relative low economic value. The main advantage of gelling starch in its natural casing is that a viscosity build-up is avoided. Because of this, the gelling in the starch-rich fraction and the release from the starch-rich fraction and the subsequent enzymatic conversion of starch can take place in relative simple devices such as a tank. Also no water has to be added to control the viscosity. Because of this, for example, an ethanol flow for the entire process can be obtained of 25 vol.% from grains of maize with 50% dry substance (DS) without intermediate water addition. At higher ethanol concentrations, the wastewater flow released is smaller as compared to other processes comprising the addition of water. This results in the advantage that less energy and less investment is needed to process ethanol and the other products.

After cooling of the gelled starch in his casing, the released starch is removed from its casing using known and common techniques, such as mechanical squeezing using a screw press or a crusher.

Subsequently, the enzymatic conversion of gelled starch into sugars takes place. For this enzymatic conversion, use can be made of classic enzymes and enzyme systems. These enzymes also comprise amylase as well as amyloglucosidases. Combined enzyme systems can also be used. It will be apparent that the possibly required thermal heat for this enzymatic conversion will be supplied from the heat force coupling.

It is also possible to add available sugars comprising residue flows to the product fermentation containing residual flows such as beet juice, grass juice, potato juice, old bread, (steamed) potato shells, calf, pig, bovine, poultry manure. In this product fermentation, the desired conversion products are formed.

The conversion products, like ethanol, which are formed by fermentation, are subsequently removed from the conversion medium and conveyed as valuable product.

The remainder of the medium comprising starch, yeast, enzymes (possibly linked to starch), such as amylase) is returned to the product fermentation. A resulting residue flow is conveyed to the biogas fermentation.

The residue fraction, as is formed from the separation of the starch-rich fraction from the starch source, can be used for generating electricity by means of direct burning as well as indirect subjecting the residue fraction as a whole or partially to biogas production (biogas fermentation) and subsequently using the biogas for generating electricity or mechanical force.

As starch source meeting the requirements of the separation of a starch-rich fraction comprising starch in its biological casing, like a bead, turnip or root, the following can be mentioned, corn, wheat, sorghum, potato, triticale, cassava, rice, sweet potato. The starch source is in fact a plant, such as a corn plant or grain plant, from which the corn grain, wheat grain or another grain, turnip, root, is separated from the remainder of the plant material which forms the residue fraction. This means that from the starch-rich fraction, a high quality end product (ethanol) can be obtained which competes at the price level of natural oil and this provides a higher economical value than the generation of energy and/or heat from this starch-rich fraction (which has to compete with coal). The less economical residue fraction can also be used for the generation of biogas which macro-economically competes with coal or natural gas. The biogas, as well the remainder fraction, can be burn in a burning device for the production of energy and heat. The advantage of using a corn plant is a relative high starch yield (8 tons per 18 tons dry matter per hectare). Hence a corn plant is the preferred starch source for the method according to the present invention.

Due to the fact that in general a starch source is not available during the whole year and the production of conversion products, in particularly ethanol, and electricity and/or heat production are desirably available during the whole year, it is preferred that the starch-rich fraction, possibly after a conservation treatment, is stored. Due to the fact that the starch-rich fraction has undergone a treatment, and can be stored during a longer period of time, this starch-rich fraction is available over the whole year for the ethanol production. Such a conservation treatment can, for example, be performed using a treatment with propanoic acid after which the starch-rich fraction can be stored, for example by means silaging. Another form of pretreatment is the increase of amount of the dry matter resulting in an improvement of the conservation. For example, by drying the amount of dry matter can be increased, another possibility is to leave the crops longer on the land.

The heating of the grain fraction is carried out in relatively simple containers. Through a heat exchanger, heat from the heat force coupling is transferred into a liquid. This heated liquid is directly contacted in the container with the starch rich fraction. Using this approach, the starch rich fraction can be heated and gelled in relatively simple containers. Because the gelling of the starch takes place in the natural casing of the starch source, no viscosity built-up takes place in the container. Because no viscosity built-up takes place, also no water needs to be added to reduce the viscosity built-up of the jelly. After a sufficient gelling of the starch in its natural casing, such as a grain, it is cooled.

Because no water needs to be added, the handling can be performed in a more concentrated way. Because of this, yeast and/or other ferments and enzymes can be used multiple times. The separation of the yeast (and/or ferments), enzymes and starch, can be performed by using a micro-sieve (1-10 µm), a centrifuge or a separator or decanter.

The heat released during this cooling can subsequently be used in the method according to the invention. The cooled starch is pressed from its natural casing, such as a grain, by for example using a screwing press or a crusher.

Preferably, the heating of the starch in its biological casing (grain) takes place at a relatively high temperature (high killing effect) causing the formed gelled starch to be in fact substantially aseptic or sterile. This high killing (preferably heat) treatment causing the microorganism present to strongly decrease in number, is in fact not a disadvantage for the subsequent microbiological and/or enzymatic conversions and provides the advantage, because during the enzymatic conversion subsequently use is made of sterile gelled starch necessitating relatively low concentrations of enzyme and yeast without an increased risk for infections. In this way, it is avoided that during the fermentation a part of the starch is not being converted into the desired conversion products desired but in undesired products.

The enzymatic conversion of gelled starch into sugars can be performed using fermentation using a classic amylase like BAN^{®}, using a classic thermostable amylase like Maxamyl^{®}, or using an enzyme which can be suitably used under the same circumstances as in yeast fermentations (a temperature between 30 and 40°C and a pH between 3 and 6), like StarGen. By using an enzyme system comprising at least one amylase and at least one amyloglucosidase, the enzymatic conversation can be performed with at a relatively high rate. This is provided because the starch is already gelled. The conversions can be performed in different containers or in a single container.

Despite what has been mentioned above, it will be clear that during the enzymatic reaction the possibly required thermal heat is derived from the heat force coupling.

Subsequent to the conversion of the gelled starch in the conversion products, such as ethanol, the conversion products are next processed from the conversion medium. This conversion can be performed using distillation, stemming or in vacuo stemming or pervastemming. Especially separation techniques which require heat for the separation. The heat is supplied by the heat force coupling. When conducting the method at a relatively small scale, use can be made for the reprocessing of ethanol and other conversion products like butanol, obtained from another reprocessing technique. This reprocessing technique comprises on one hand gas stemming followed by ethanol condensation. The gas stemming of ethanol or butanol is provided by conveying a gas through the conversion medium. Because of the volatility of ethanol, ethanol will be conveyed by this gas flow leaving the conversion medium. It will be understood that depending on the process pressure, temperature, etc, as also the composition of the conversion medium, ethanol, in a certain amount, can be removed through gas stemming. Important for this are the liquid-vapour balance of water and the accompanying ethanol volatility. For a skilled person, it will be apparent which process conditions must be used to provide an optimal gas stemming of ethanol and other conversion products such as butanol from the conversion medium. The gas stemming can be provided using any type of inert gas (the volatility of ethanol is hardly dependent on other gases in the gas phase). A CO₂ stemming is preferred and in particularly CO₂ derived from the fermentation of sugars into ethanol and CO₂. An additional advantage is that, after the CO2 stemming, the remainder of the CO₂ can be further used in horticulture and because of this can be recycled and reused for fertilization in glass houses.

In case of vacuum stemming, the volatile conversion products (for example ethanol) and water are separated under a reduced pressure in a gas stemming column. After compression, the vapor is cooled and condensated.

For a continuous electricity and ethanol production, it is also desired that a continuous adequate amount of residual fraction is available. Because of this, it is preferred that the residual fraction, after harvest and separation, is stored by for example silaging. For biogas generation, less biomass is available because of the earlier separation of the starch-rich fraction from the starch source. For compensation of the removal of easily fermentated components from the raw material for the fermentor, it is preferred to provide that the residual fraction is readily available for the production of biogas. In this way, still sufficient biogas is available for existing installations for heat force coupling allowing for a placement of the ethanol production unit. De pretreatment can particularly be performed such as to provide a better use of the cellulose and lignocellulose present in the residual fraction. Of course, when using newly designed processing installations, a high as possible yield of all products is prefered. In this context, a pretreatment is envisaged as described in Maas R.H.W., Thesis: Microbial conversion of lignocellulose-derived carbohydrates into bioethanol and lactic acid. Chapter 2: Mild-temperature alkaline pretreatment of wheat straw to enhance hydrolysis and fermentation, 2008, in Klaasse Bos, G.J., Thermochemical pre-treatment of lignocellulosic biomass to enhance anaerobic biodegradability, 2007, and in Klaasse Boss G-J. Optimalisatie van de biogaswinning uit organische reststromen, 2007. A pretreatment by means of calcium hydroxide 10%, on dry solid base, a heating from 7 °C-10 °C is preferred considering the improvement of the yield of biogas obtained from the residual fraction.

The digest from the biogas fermentation can be separated in a thin and thick fraction. The thick fraction from the biogas fermentation still comprises notable amounts of undigested cellulose and lignocellulose. By a first heat step, a large part of the dissolved CO₂ will evaporate. In the next treatment, because of this, the amount of Ca(OH)₂ ,which is required to achieve a sufficient high pH, is decreased. By adding Ca(OH)₂ ammonia will be released. The advantage of this is that the amount of nitrogen will decrease. Because of this, more digest can be brought on the land without violating the environmental directives. The ammonia can be separated by using an aqueous solution of acid, such as for example sulphuric acid. Also the CO₂ released during the heating and the ammonia obtained by adding Ca(OH)₂ can be brought in contact with each other in a moist environment. This can yield ammonium carbonate or ammonium bicarbonate. Using this, ammonia can be concentrated in a relatively small volume without using an acid washing liquid. In case insufficient CO₂ is released during the heating, also CO₂ from the fermentation can be used. Reconveying the treated digest in the biogas fermentation provides additional raw material for the biogas fermentation.

Also a yeast residue flow from the product fermentation can be conveyed to, and be used in, the biogas fermentation.

Another aspect according to the present invention relates to a device for carrying out the previously described methods for the production of electricity and ethanol. This device comprises
- a separation unit for separating a starch source into a starch-rich fraction and a residue fraction;
- a heating unit for heating of the starch-rich fraction for the gelling of starch;
- a unit for releasing of the gelled starch;
- at least one enzymatic conversion unit for the gelled starch into sugars and for the conversion by fermentation of sugars into conversion products;
- a production unit for the production of biogas from the residue fraction;
- coupled heat force unit for the combined production of electricity or mechanical energy and heat from the residue fraction and/or the biogas;
- a production unit for reprocessing conversion products; and
- heat supplying means for the heat supply from the heat producing unit to at least one of the other units from the device.

It will be appreciated that when using the device according to the invention, and using relatively simple equipment and using standard techniques and heat force coupling, in a relatively simple and economic feasible way at a small scale (for example one MWₑ) electricity and conversion products can be produced from a specific starch source which is only available during a part of the year. By the specific treatments according to the invention, the starch source is made available during the entire year.

The method and device according to the invention are also suitable for use at a relatively small scale. Because of this, it is also possible to use the remaining fibers and minerals in agriculture because of the relatively low transport costs. Because of this, the method and device according to the invention are suitable for a relative fast implementation of the method and device in an existing infrastructure.

The indicated and other features of the method and device according to the invention will further be elucidated using an embodiment which is only provided by way of an example without being limiting for the scope of the invention. In this embodiment, reference is made to the following drawings wherein:
Figure 1 is a flow diagram of a method and device according to the invention;
Figure 2 shows an overview of an embodiment of a method according to the invention, whereby stripe-arrows mass flows and dot-arrow heat flows are illustrated;
Figure 3 shows a flow diagram of a pretreatment of the starch-rich fraction and the subsequent ethanol fermentation and distillation;
Figure 4 show a flow diagram of the treatment of the digest from the biogas fermentation;
Figure 5 shows a comparison of the CO₂ (ethanol) production for glucose (___ and _ ..._), corn starch (_.._ and _._) and released gelled starch (---) as prepared according to the invention;
Figure 6 shows the CO₂ (ethanol) production rate; and
Figure 7 shows the increase of the glucose concentration in the presence of enzymes and the absence of yeast.

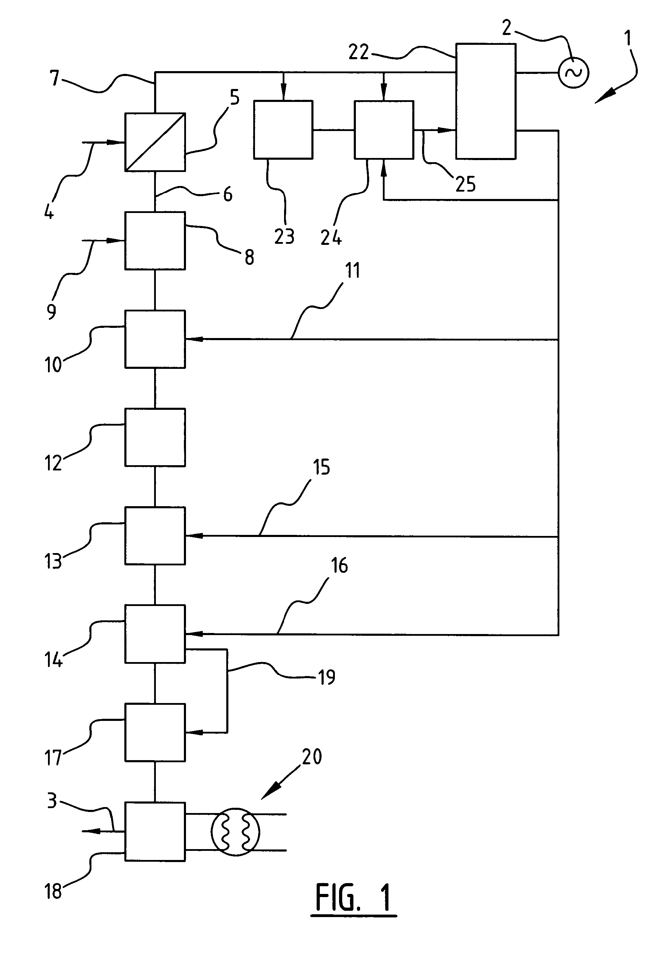

Figure 1 schematically shows a device 1 according to the invention for the production of electricity 2 and ethanol 3.

To the device, through arrow 4, a starch source is supplied. This starch source can comprise corn plants, grain plants, like wheat plants, etc. Through arrow 4, the starch source, in this case corn plants, arrive in a unit 5 for the separation of the starch source into a starch-rich fraction 6 and a residue fraction 7. In the separation unit 5, a separation can be provided between corn grain and corn plant parts, using, for example, a separation technique as is used for bovine feeding.

The corn grains are subsequently conveyed through a storage unit 8 wherein the corn grains desirably can be stored during an extended period of time before being used for further processing. In the storage unit 8, through arrow 9, a conservative can be added. In this case, for example, proponic acid be added. In fact, the conservative is only used for the protection of corn grains against an undesirable early disintegration, whilst the conservative does not provide any adverse effects, particularly in the subsequent enzymatic and biological conversion.

After storing in storage unit 8, the corn grains are conveyed to a heating unit 10 which in fact comprises a container supplied with a heat exchanger to which heat, through arrow 11, is conveyed. In the heating unit 10, the corn grains, using hot water, are heated to, for example, a temperature of 60 °C to 120 °C. This temperature is chosen to provide gelling of the starch in the corn grain, but not a release from the corn grains. By this, an increase in viscosity in the heating unit 10 is avoided and, because of this, the heating unit can be of relative simple design. The heating is preferably performed in such a way that it has a high killing activity on the gelled starch which is substantially sterile (a relatively low plate count).

After a sufficient heating for gelling of the starch into gelled sterile starch, the starch is subsequently conveyed to unit 12, wherein, using mechanical means, the gelled starch is released from the corn grain. The unit 12 can, for example, comprise a toothwheel pump, a screwing press or another type of press. In this context, it is important that for the release of the gelled sterile starch, the starch remains as much as possible sterile and is suitable to be directly supplied to the subsequent units for the enzymatic conversion of the gelled starch which has been removed from the starch grain.

The enzymatic conversion of the sterile, gelled starch occurs in unit 13. Unit 13 provides an enzymatic conversion of the sterile starch into glucose by means of digestion enzymes, such as amylase and glucamylase. The glucose formed is subsequently conveyed to unit 14 in which the glucose is converted, by yeast fermentation, into ethanol.

As indicated in Figure 1, heat is applied through arrow 15 and arrow 16 to the units 13 and 14, respectively (if desired), to provide optimal circumstances for the conversion of starch, via glucose, into ethanol.

The formed ethanol is subsequently conveyed to a unit 17 for the processing of the produced ethanol. Unit 17 provides a CO₂ stemming in which the ethanol present in the conversion medium is separated by stemming using CO₂. This ethanol containing CO₂ flow is conveyed to unit 18. The for the CO₂ stemming required CO₂ is derived from the fermentation unit 14, wherein the glucose is converted into ethanol and amongst others, CO₂. This CO₂ is conveyed by means of a by-pass pipe to the gas stemming device of the processing unit 17. The remainder of the conversion medium can be recycled (because the supplied gelled starch was sterile) as also used for the production of biogas for direct use for the production of energy. The unit 18 comprises a condensation unit for condensation of ethanol from the CO₂ gas flow which can be re-supplied to the gas stemming device. The condensed ethanol has in general a concentration of 10 to 60 vol.%, for example 15 to 35 vol.%, like 17 vol.% In general, such a concentration can be provided that a good preservation is obtained (from approximately 15 vol.% to 99 vol%).

This ethanol can serve as raw material for an ethanol plant for the processing of ethanol to a higher concentration and accompanying specification. The provided ethanol fraction can comprise traces of organic compounds like organic acids, which, for the moment, need not to be removed from the device 1 according to the invention.

Figure 1 further shows that remainder fraction 7 can directly be added to a burner unit 22 for the generation of electricity 2. It is however preferred that, as shown in Figure 1, the residue fraction is conveyed to a storage unit 23 from which, if desired, the residue fraction can directly be added to the burner unit 22 of the heat force coupling. For an optimal use of the residue fraction, it is however preferred to convey the residue fraction to a pre-treatment unit 23 wherein the residue fraction is further pre-treated for a better use of the lignocellulose present in the residue fraction in a therewith connected unit 24 for the production of biogas. It will be clear that the to the biogas unit a residue fraction which has been pretreated or not has been treated can be supplied. The generated biogas can through arrow 5 directly be supplied to the burner of the heat force coupling 22. In the heat force coupling, combined electricity and mechanical/thermal energy is generated. The thermal energy can, as shown in Figure 1, be used in a several units and in particularly also in the biogas unit 25. Although not shown in Figure 1, it will be clear that other residue fractions from the method and device according to the invention can also be added to the biogas unit as well as other raw material flows as manure and other agricultural residue products for a further optimal use in the biogas unit 24.

Not shown is that a residue fraction from the product fermentation in unit 14 can be conveyed to the biogas fermentation in unit 24.

As is clearly shown in Figure 1, it is possible, at a relative small scale, and using a relatively simple unit, to economically produce energy and the heat formed by this, via heat force coupling, for the production of bio fuel, the pretreatment of the residue fraction, the treatment of the digest and the production of biogas. Further, optimal use of the residue heat is provided.

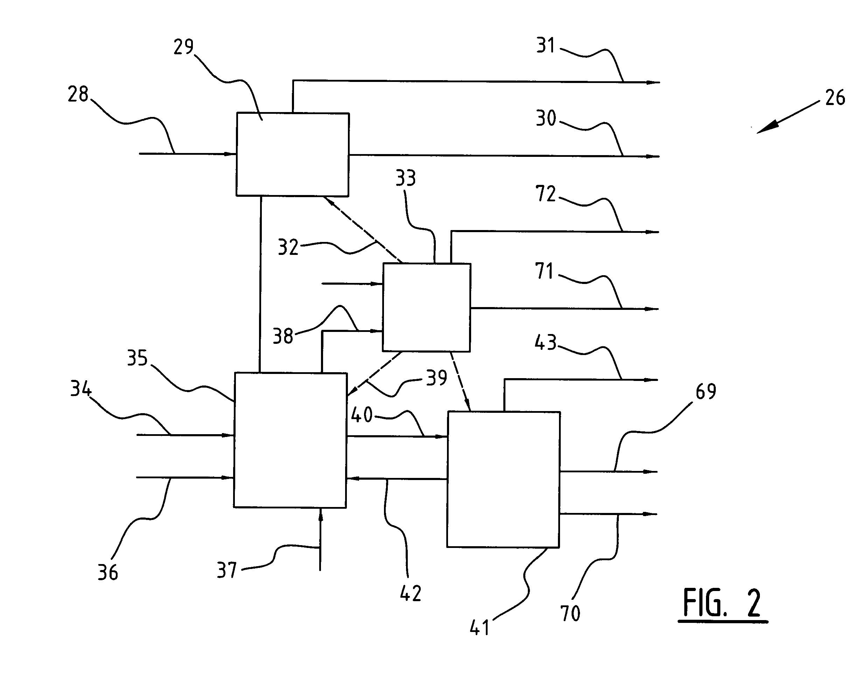

Figure 2 shows an overview of a method and device 26 according to the invention. The starch rich fraction (corn grains) is conveyed through pipe 28, to the product fermentation in unit 29. In unit 29, the enzymatically released, gelled starch is converted into sugars and subsequently into ethanol. Ethanol is separated through conduit 30 and the CO₂ formed during the fermentation is separated through conduit 31. The heat required for particularly the processing of ethanol, is, via conduit 32 supplied from the heat force coupling 33.

The residue fraction of the corn plants is, through conduit 34, conveyed to a unit 35 for the fermentation of biogas. Through conduit 36 manure is conveyed and through conduit 37 the residue flows and optionally a residue fraction from the product fermentation unit 29. Through conduit 38, biogas is supplied to the heat exchange coupling 33. The heat required for the biogas production is supplied from the heat force coupling 33 as indicated by arrow 39. Digest from the biogas fermentation 35 is, through conduit 40, conveyed to a unit 41 for a treatment of the digest and particularly digestion of the non-digested cellulose and lignocellulose present, which are, through conduit 42 reconveyed to the biogas fermentation unit 35. The CO₂ dissolved in the digest, and ammonia, are separated through the conduit 43. Wastewater is separated through the conduit 69 and solid matter (especially sand/lignin), are separated through conduit 70.

Generated electricity by means of heat force coupling 33 is supplied, via a conduit 71, to the power grid and residue gas is separated via conduit 72.

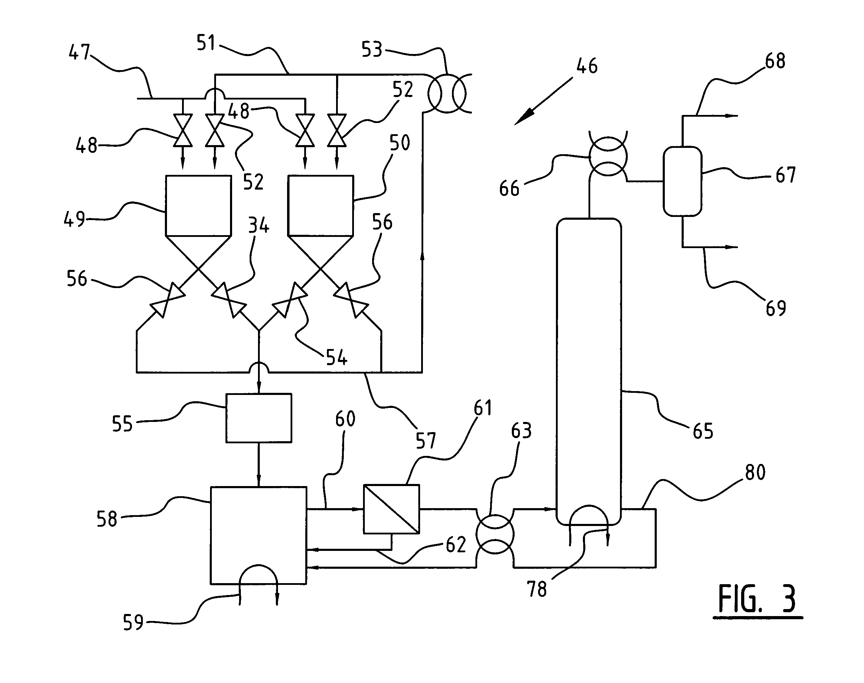

Figure 3 shows a device 46, forming part of the device 26 shown in the flow diagram of Figure 2 according to the invention. A starch-rich fraction, corn grains 47, are in parallel, via closings 48, supplied to a silo 49, 50. In this silo 40, 50 hot water 51 is supplied through the valves 52, and sprayed on the corn grains. Hot water 51 has a temperature of approximately 100 °C and is heated in a heat exchanger 53 to which heat is supplied from a non-shown heat force coupling. After sufficient gelling, the corn grains are alternating from silo 40 of 50, via valve 54, conveyed to the opening device 55.

During gelling, water which is sprayed on the starch grains, can be separated after a sufficient incubation time via valves 56 and via a conduit 57 through the heat exchanger 53 and reconveyed to silo's 49, 50.

In the release unit 55, using for example a crusher, the gelled starch is released from its biological casing and carried to a unit 58 for the enzymatic conversion of the gelled starch into sugars and, subsequently, into ethanol by fermentation. At this point, it is noted that from the treatment with hot water to the arrival at unit 58, due to the killing activity of the hot water, the number of microorganisms present is dramatically decreased to such a level that this plays no role during the enzymatic starch conversion and sugar fermentation.

Unit 58 can not only comprise one conversion reactor, but also one or more reactors. For example, in a first reactor, using Maxamyl^{®} at 90 °C and a pH of 7, starch can be converted. Subsequently, in a subsequent unit, at a pH of 4.5 and a temperature decreasing from 60 °C to 35 °C using aminoglucosidase and yeast, the conversion, through sugar, into ethanol can be provided. Using the heat exchanger 59, if necessary, heating or cooling can be performed.

The conversion medium from the unit 58 will be conveyed via conduit 60, to a separator 61, such as a micro sieve (more than 0.5µm, like 1-5µm), a centrifuge or separator/decanter). The separated starch/yeast is reconveyed, via a conduit 62, to unit 58. The remainder of the solid phase is conveyed, via a heat exchanger, to a distillation column 65, in which, at atmospheric pressure via only a limited number of distillation dishes, 78 vol.% of ethanol is distillated via heat exchanger 66, while the supply stream comprises 7.5 vol.% of ethanol. Via separator 67, CO₂, through conduit 68, is separated and ethanol, via conduit 69. A bottom stream 70, which is obtained after passing one or more distillation dishes, is via heat exchanger 53 re-conveyed to unit 58. The heat required for use of the distillation column 65 is supplied from the heat force coupling via the heat exchanger 78. A bottom fraction of the distillation column 65 is re-conveyed through a conduit 80 and a heat exchanger 63 to the fermentation unit 58.

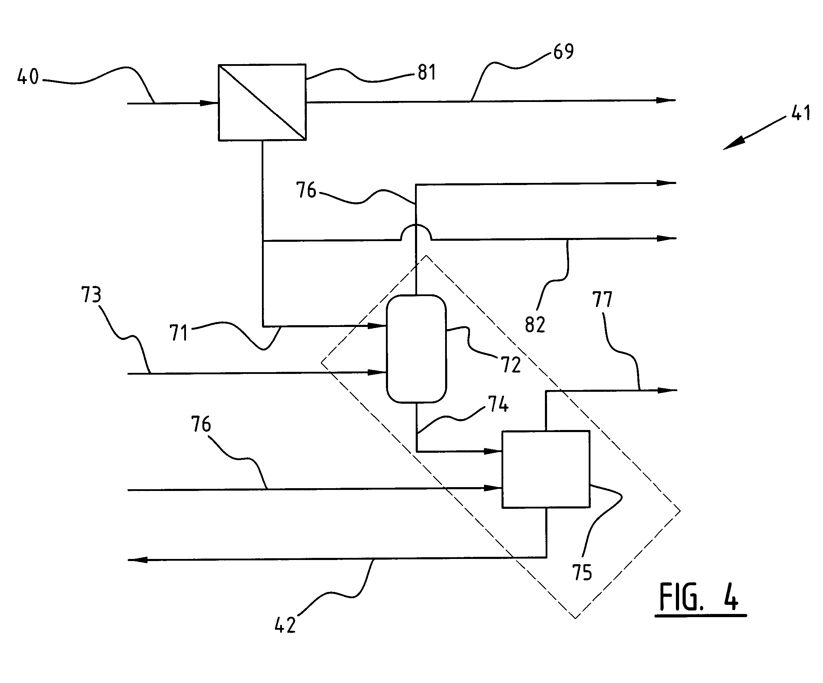

Figure 4 shows in more detail the unit 41 for the treatment of the digest. The digest is conveyed, through the conduit 40, to a separation unit 81. Wastewater (or thin fraction) is conveyed through the conduit 69. The residue flow is divided in such a way that, through the conduit 82, a solid mass (sand/lignin) is separated. The remainder part (or thick fraction) is, through a conduit 71, conveyed to a heating/degassing unit 72. Degassing is carried out using steam which is supplied from the heat force coupling 34 through conduit 73. CO₂ and biogas are separated through conduit 76. The degassed thick fraction comprising cellulose/lignocellulose is, through conduit 74, conveyed to the treatment tank 75 wherein to the hot cellulose/lignocellulose (approximately 85 °C) calcium hydroxide is added through conduit 76. Units 72 and 75 are optionally combined. The ammonia formed is separated through the conduit 76 and can be recycled by washing. After an incubation at this high temperature, the treated cellulose/lignocellulose, through conduit 76, is re-conveyed to the biogas fermentation unit 35 after approximately one day.

In supplementary experiments, it has been investigated if the gelled and released starch derived from the starch-rich fraction is suitable for the conversion of ethanol by fermentation. For this, a comparison is made with other carbon sources and particularly glucose and corn starch.

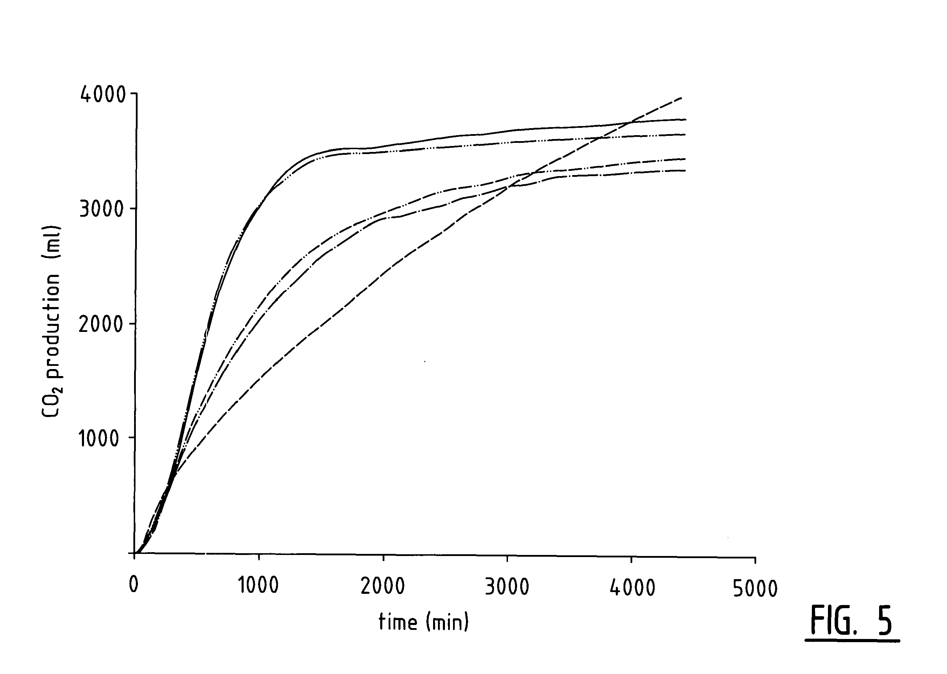

Figure 5 shows that, in time, the CO₂ production (equivalent to ethanol formation) is similar to the CO₂ /ethanol production based on glucose and corn starch.

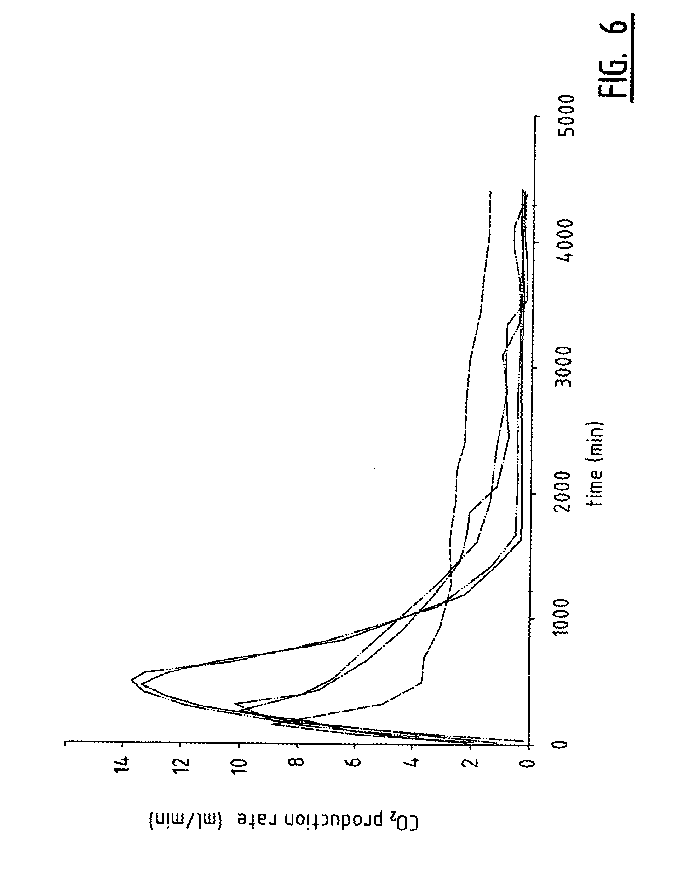

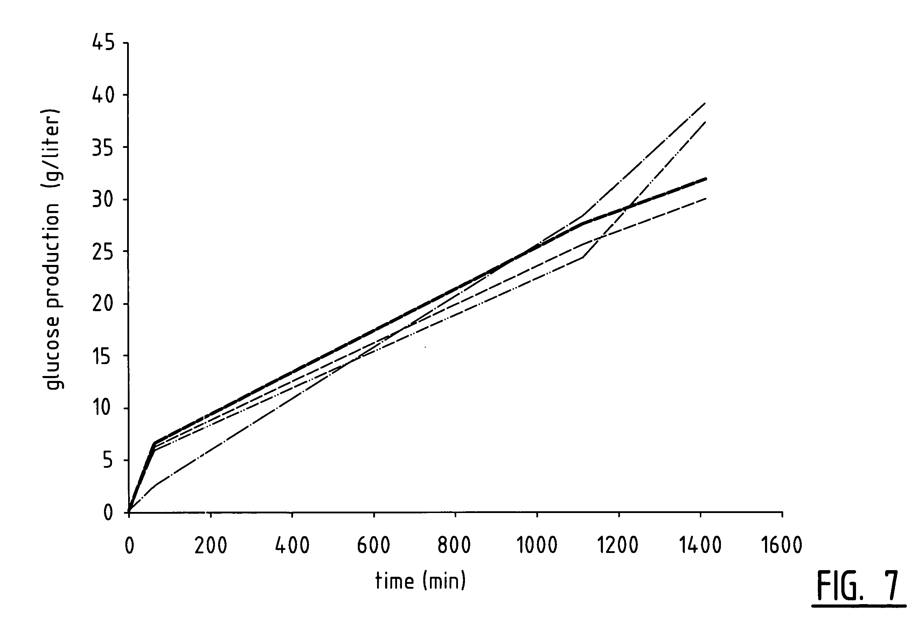

Figure 6 shows the CO₂/ethanol production rate and Figure 7 shows the accompanying glucose concentration in time after addition of the enzymes.

The above results show that gelled and released starch from the starch-rich fraction, by using enzymes, can be adequately converted into sugar and subsequently into ethanol.

Although the embodiments are only discussed in relation to the production of ethanol as conversion products, it will be clear that by using other enzymes and microorganisms and/or the use of other conditions, other desired conversion products or combinations thereof can be provided using the method and device according to the invention.

## Claims

1. Method for the production of electricity and conversion products, such as ethanol, comprising the steps of:
i) separating a starch source into a starch-rich fraction and a residue fraction;
ii) heating of the starch-rich fraction for the gelling of starch;
iii) releasing of the gelled starch from the starch-rich fraction;
iva) enzymatically converting the gelled starch into sugars;
ivb) converting the sugars into conversion products by fermentation;
v) processing conversion products from the conversion medium;
vi) producing biogas from the residue fraction;
vii) producing electricity and heat from the biogas and/or the residue fraction using heat force coupling; and
viii) using heat produced for one or more of the steps i) to vi).

2. Method according to claim 1, wherein the starch-rich fraction, optionally after a conservation treatment, is stored.

3. Method according to claim 1 or 2, wherein the steps ii), iii) and iv) are substantially performed under aseptic circumstances.

4. Method according to any of the claim 1 to3, wherein the gelled starch from the starch-rich fraction is pressed.

5. Method according to any of the claims 1 to 4, wherein the enzymatic conversion comprises an enzymatic conversion of starch into sugars and a conversion of the sugars into conversion products, such as ethanol by fermentation.

6. Method according to claim 5, wherein use is made of an enzyme system which at least comprises one amylase and at least one amyloglucosidase.

7. Method according to any of the claims 1 to 6, wherein the enzymes for enzymatic conversion and/of ferments for the conversion by fermentation are retained.

8. Method according to any of the claims 1 to 7, wherein step v) comprises ethanol distillation.

9. Method according to any of the claims 1 to 8, wherein in step v) ethanol is produced by means of gas stemming and ethanol condensation.

10. Method according to claim 9, wherein the gas stemming comprises gas stemming of CO₂.

11. Method according to claim 10, wherein CO₂ is derived from step iv).

12. Method according to any of the claims 1 to 11, wherein the heating of the starch-rich fraction in stap ii) is carried out using heat generated in the heat force coupling.

13. Method according to any of the claims 1 to 12, wherein the residue fraction is stored.

14. Method according to any of the claims 1 to 3, wherein the residue fraction and/or thick fraction from the biogas fermentation is treated, for example using calcium hydroxide.

15. Method according to claim 14, wherein the thick fraction is heated for removal of dissolved carbon dioxide.

16. Method according to any of the claims 1 to 15, wherein the starch source comprises corn.

17. Method according to any of the claims 1 to 16, wherein the ammonia formed is removed, for example using carbon dioxide formed.

18. Device for carrying out the method according to any of the claims 1 to 17, comprising:
- a unit for separating a starch source into a starch-rich fraction and a residue fraction;
- a unit for heating of the starch-rich fraction for gelling of the starch;
- a unit for the release of gelled starch from the starch-rich fraction;
- at least one unit for the enzymatic conversion of gelled starch into sugars and for the conversion of sugars into conversion products by fermentation;
- a unit for producing biogas from the residue fraction;
- a coupled heat force unit for the combined production of electricity or mechanical energy and heat from the residue fraction and/or the biogas;
- a unit for the processing of the conversion products; and
- means for conveying heat from the heat force coupling unit to at least one of the other units from the device.

19. Device according to claim 18, comprising a unit for the storage of the starch-rich fraction and/or the residue fraction.

20. Device according to claim 18 or claim 19, wherein the unit for the enzymatic conversion comprises a first container for the enzymatic conversion of starch and a second fermentation container for the conversion to sugars and ethanol.

21. Device according to any of the claims 18 to 20, wherein the unit for the treatment of the conversion products comprises an ethanol distillation.

22. Device according to any of the claims 18 to 21, wherein the unit for the treatment of ethanol comprises a gas stemming device and an ethanol condensation unit.

23. Device according to any of the claims 18 to 22, comprising a unit for the treatment of the residue fraction and/or a thick fraction from the conversion by fermentation.
